# EUROPEAN PATENT SPECIFICATION

(11) **EP 1 679 037 B1**
(45) Date of publication and mention of the grant of the patent: **20.08.2008**
(21) Application number: 05020046.8
(22) Date of filing: 15.09.2005
(51) Int. Cl.: A61B 8/00, G06F 19/00, G01S 7/52

(54) **Client/server-based ultrasound diagnostic system**
Kunden-Server-basiertes Ultraschalldiagnosesystem
Système de diagnostic à ultrasons sur une base client-serveur

(30) Priority: 07.01.2005 KR 2005001681
(43) Date of publication of application: 12.07.2006
(73) Proprietor: MEDISON CO., LTD., Kangwon-do 250-870 (KR)
(72) Inventor: Hyun, Dong Gyu, Gwangju-si, Gyeonggi-do 464-725 (KR)
(74) Representative: Schmid, Wolfgang

(56) References cited:
- US-A1- 2002 007 119
- US-A1- 2002 040 186
- US-A1- 2003 115 018

## Description

The present invention generally relates to an ultrasound diagnostic system, and more particularly to a client/server-based ultrasound diagnostic system.

An ultrasound diagnostic system is known as medical equipment for obtaining information on the internal organs of the human body in a non-destructive manner.
The ultrasound diagnostic system operates to irradiate an ultrasound signal through the surface of the human body to the selected internal organ and receives the echo signal reflected from the internal organ. The echo signal is then processed to provide, for example, a cross-sectional image of a soft tissue or bloodstream within the human body.

As is well known, the ultrasound diagnostic system is smaller in size and is cheaper compared to other image diagnostic devices (e.g., X-ray diagnostic device, X-ray computer tomography (CT) scanner, magnetic resonance imaging (MRI), nuclear medicine diagnostic device, etc.). The ultrasound diagnostic system can safely provide a bloodstream image since it does not require the human body to be exposed to an X-ray.

A conventional ultrasound diagnostic system generally consists of three parts: a front end for receiving ultrasound signals; a back end for processing the received ultrasound signals; and a host processor in charge of controlling the overall system in response to the user control and for displaying the results of the processing. However, the conventional system is disadvantageous since its parts are not replaceable. That is, even if one part of the system has been upgraded in terms of function, the user must newly purchase the entire system as upgraded if he/she wishes to utilize the upgraded function. For example, if only the function of the back end has been upgraded, the user must purchase a new ultrasound diagnostic system in order to utilize the upgraded function of the back end, even though the functions of the front end and the host processor remain the same as the old system. Further, given that a processor of the host processor is continuously improved, it would be disadvantageous for a user to purchase a new ultrasound diagnostic system whenever a newly developed processor is adopted.

Another disadvantage of the conventional system is that the required functions of the ultrasound diagnostic system may vary depending on a particular medical field. Therefore, it would be disadvantageous to develop an ultrasound diagnostic system specific for each medical field.

It is an objective of the present invention to provide a client/server-based ultrasound diagnostic system that allows the functions of each respective unit to be individually upgraded, as well as being adapted to control a plurality of front ends simultaneously.

In accordance with the present invention, there is provided a client/server-based ultrasound diagnostic system, comprising: a plurality of clients, each client comprising a probe for transducing an electric signal into an ultrasound signal, and vice versa, a beam former for transceiving the electric signal from/to the probe, and a display unit; and a server connected to the clients for controlling the probe and the beam former on each client, generating a corresponding ultrasound image signal based on the electric signal received from each respective client, and transmitting the corresponding generated ultrasound image signal to the display unit of each respective client.

In accordance with another aspect of the present inventioneach client comprises an analog/digital converter and its beam former comprises a pulse signal generating unit for generating a plurality of electric pulse signals, a transmit beam forming unit for forming a transmit beam by delaying the electric pulse signals received from the pulse signal generating unit, and a receive beam forming unit for forming a receive beam by delaying the electric pulse signals received from the probe wherein the analog/digital converter converts the receive beam into a digital receive signal and the server comprises a digital scan converter for forming corresponding image data based on the digital receive signal received from the analog/digital converter of each respective client and an image processor for processing the corresponding formed image data received from the digital scan converter for transmission of the corresponding processed image data to the display unit of each respective client.

The above and other objects and features of the present invention will become apparent from the following description of preferred embodiments provided in conjunction with the accompanying drawings, in which:
Fig. 1 is a block diagram illustrating a client/server-based ultrasound diagnostic system in accordance with the present invention; and
Fig. 2 is a block diagram illustrating a beam former of Fig. 1.

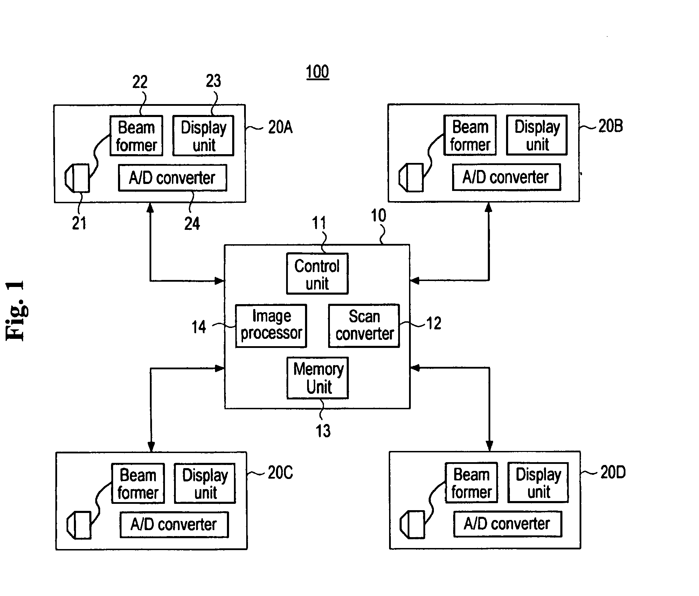

Fig. 1 is a block diagram illustrating a client/server-based ultrasound diagnostic system in accordance with the present invention.

Referring to Fig. 1, the client/server-based ultrasound diagnostic system 100 includes a server 10 and a plurality of clients 20A, 20B, 20C, 20D connected to the server 10. The server 10 may receive and transmit data from/to the clients 20A, 20B, 20C, 20D via a network protocol such as TCP/IP, UDP/IP, ATM, etc. For example, the clients 20A, 20B, 20C, 20D may be arranged in an obstetrics department, a gynecology department, an internal treatment department, etc. of a hospital.

The server 10 includes a control unit 11, a scan converter 12, a memory unit 13 and an image processor 14. The respective clients 20A, 20B, 20C, 20D include a probe 21, a beam former 22 and a display unit 23.

The probe 21 includes a transducer, a matching layer and a backing layer. The probe 21 may have one transducer or a linear transducer array that is comprised of a plurality of transducers (i.e., one-dimensional transducer array). The transducer converts an electrical pulse signal into an ultrasound signal, as well as converting an ultrasound signal into an electrical pulse signal. The matching layer corrects a sound difference between a human body and an oscillator in the probe 21. The backing layer absorbs the sound energy of the oscillator to form an electrical pulse signal.

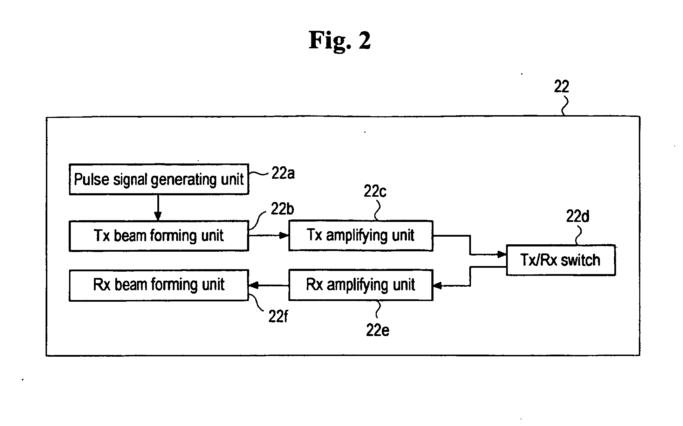

Fig. 2 is a block diagram illustrating a beam former of Fig. 1.

Referring to Fig. 2, the beam former 22, which is arranged in the clients 20A, 20B, 20C, 20D, comprises: a pulse signal generating unit 22a; a transmit beam forming unit 22b; a transmit beam amplifying unit 22c; a transmit/receive switch (Tx/Rx switch) 22d; a receive beam amplifying unit 22e; and a receive beam forming unit 22f.

Under the control of the control unit 11, which is arranged in the server 10, the pulse signal generating unit 22a generates a plurality of transmit pulse signals and then transmits those signals to the transmit beam forming unit 22b. The transmit beam forming unit 22b receives the plurality of transmit pulse signals and then forms a transmit pattern. That is, the transmit beam forming unit 22b may delay the respective transmit beam signals according to the control of the control unit 11 so as to form a transmit pattern. The transmit beam amplifying unit 22c amplifies the transmit pulse signals received from the transmit beam forming unit 22b. The Tx/Rx switch 22d transmits the amplified transmit pulse signals to the probe 21. The transducer or the transducer array arranged in the probe 21 converts the transmit pulse signals into an ultrasound signal. The ultrasound signal is then transmitted to the target object.

The transducer or the transducer array converts echo signals reflected from the target object into an electrical pulse signal. The Tx/Rx switch 22d and the receive beam amplifying unit 22e transmit the converted electrical pulse signal to the receive beam forming unit 22f. Considering that respective phases of the echo signals are different according to a distance between the target object and the transducer (or the transducer array), the receive beam forming unit 22f delays the respective electrical pulse signals to match the phases of the electrical pulse signals with one another. It then sums up the electrical pulse signals having the equal phase to form a receive beam. An analog/digital converter 24 converts the receive beam into a digital receive signal and then transmits the digital receive signal to the server 10.

The digital scan converter 12, which is arranged in the server 10, receives the digital receive signal, sequentially scans an image corresponding to the location of the transducer or the transducer array, and stores image data into the memory unit 13. According to the control of the control unit 11, the digital scan converter 13 reads the image data stored in the memory unit 13 to transmit the read image data into the image processor 14. The image processor 14 processes the image data transmitted from the digital scan converter 12, generates an ultrasound image signal, and transmits the ultrasound image signal to the display unit 22 arranged in the clients 20A, 20B, 20C, 20D.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. A client/server-based ultrasound diagnostic system, comprising:
a plurality of clients (20A, 20B, 20C, 20D), each client comprising a probe (21) for transducing an electric signal into an ultrasound signal, and vice versa, a beam former (22) for transceiving the electric pulse signal from/to the probe, and a display unit (23); and
a server (10) connected to the clients for controlling the probe and the beam former of each client generating a corresponding ultrasound image signal based on the electric signal received from each respective client, and transmitting the corresponding generated ultrasound image signal to the display unit of each respective client.

2. The client/server-based ultrasound diagnostic system of Claim 1,
wherein each client further comprises an analog/digital converter (24), and wherein the beam former comprises a pulse signal generating unit (22a) for generating a plurality of electric pulse signals, a transmit beam forming unit (22b) for forming a transmit beam by delaying the electric pulse signals received from the pulse signal generating unit, and a receive beam forming unit (22f) for forming a receive beam by delaying the electric pulse signals received from the probe, and the analog/digital converter converts the receive beam into a digital receive signal,
wherein the server comprises:
a digital scan converter (12) for forming corresponding image data based on the digital receive signal received from the analog/digital converter at each respective client, and
an image processor (14) for processing the corresponding formed image data received from the digital scan converter for transmission of the corresponding processed image data to the display unit of each respective client

## Patentansprüche

1. Kunden-/Server-basiertes Ultraschalldiagnosesystem, welches Folgendes aufweist:
eine Vielzahl von Kunden (20A,20B,20C,20D), wobei jeder Kunde einen Messgeber (21) zum Umwandeln eines elektrischen Signals in ein Ultraschallsignal und umgekehrt, einen Strahlformer (22) zum Senden und Empfangen des elektrischen Pulssignals von/zu dem Messgeber und eine Anzeigeeinheit (23) aufweist; und
einen mit den Kunden verbundenen Server (10) zum Steuern des Messgebers und des Strahlformers jedes Kunden zum Erzeugen eines entsprechenden Ultraschallabbildungssignals basierend auf dem von dem jeweiligen Kunden empfangenen elektrischen Signal und zum Übertragen des entsprechenden erzeugten Ultraschallabbildungssignals zu der Anzeigeeinheit des jeweiligen Kunden.

2. Kunden-/Server-basiertes Ultraschaldiagnosesystem nach Anspruch 1, wobei jeder Kunde des weiteren einen Analog/Digitalwandler (24) aufweist, und wobei der Strahlformer eine Impulssignalerzeugungseinheit (22a) zum Erzeugen einer Vielzahl von elektrischen Impulssignalen, eine Übertragungsstrahl-Erzeugungseinheit (22b) zum Erzeugen eines Übertragungsstrahls durch Verzögern der von der Impulssignalerzeugungseinheit empfangenen elektrischen Pulssignale und eine Empfangsstrahlbildeeinheit (22f) zum Bilden eines Empfangsstrahls durch Verzögern der von dem Messgeber empfangenen elektrischen Impulssignale aufweist, und wobei der Analog/Digitalwandler den empfangenen Strahl in ein digital empfangenes Signal umwandelt,
wobei der Server Folgendes aufweist:
einen Digital-Scan-Wandler (12) zum Bilden entsprechender Abbildungsdaten basierend auf dem digitalen Empfangssignal, die von dem Analog/Digitalwandler des jeweiligen Kunden empfangen wurden, und
eine Bildverarbeitungseinrichtung (14) zum Verarbeiten der entsprechend gebildeten Abbildungsdaten, die von dem digitalen Scanwandler zum Übertragen der entsprechenden verarbeiteten Abbildungsdaten zu der Anzeigeeinheit des jeweiligen Kunden empfangen wurden.

## Revendications

1. Système de diagnostic à ultrasons sur une base client-serveur, comprenant:
une pluralité de clients (20A, 20B, 20C, 20d), chaque client comprenant une sonde (21) pour capter un signal électrique dans un signal ultrasonique, et vice versa, un dispositif de formation de faisceau (22) pour l'émission-réception du signal à impulsions électriques de et vers la sonde, et une unité d'affichage (23); et
un serveur (10) relié aux clients pour commander la sonde et le dispositif de formation de faisceau de chaque client, générer un signal d'image ultrasonique correspondant sur la base du signal électrique reçu de chaque client respectif, et transmettre le signal d'image ultrasonique généré à l'unité d'affichage de chaque client respectif.

2. Système de diagnostic à ultrasons sur une base client-serveur de la revendication 1,
dans lequel chaque client comprend en outre un convertisseur analogique/numérique (24), et dans lequel le dispositif de formation de faisceau comprend une unité de génération de signal à impulsions (22a) pour générer une pluralité de signaux à impulsions électriques, une unité d'émission de formation de faisceau (22b) pour former un faisceau d'émission en retardant les signaux à impulsions électriques reçus de l'unité de génération de signaux à impulsions et une unité de réception de formation de faisceau (22f) pour former un faisceau de réception en retardant les signaux à impulsions électriques reçus de la sonde, le convertisseur analogique/numérique convertissant le faisceau de réception en un signal de réception numérique,
dans lequel le serveur comprend:
un convertisseur numérique (12) pour former des données image correspondantes sur la base du signal de réception numérique reçu du convertisseur analogique/numérique de chaque client respectif, et
un processeur d'image (14) pour traiter les données image formées correspondantes reçues du convertisseur numérique pour la transmission des données image traitées correspondantes à l'unité d'affichage de chaque client respectif.
